# EUROPÄISCHE PATENTSCHRIFT

(11) **EP 2 713 996 B1**
(45) Veröffentlichungstag und Bekanntmachung des Hinweises auf die Patenterteilung: **17.10.2018**
(21) Anmeldenummer: 12727131.0
(22) Anmeldetag: 31.05.2012
(51) Int. Cl.: A61K 8/34, A61K 8/81, A61K 8/06, A61Q 19/00

(54) **EMULSIONSZUBEREITUNGEN MIT VERBESSERTEN RHEOLOGISCHEN EIGENSCHAFTEN**
EMULSION PREPARATIONS WITH ENHANCED RHEOLOGICAL PROPERTIES
PRÉPARATIONS ÉMULSIFIÉES AYANT DES PROPRIÉTÉS RHÉOLOGIQUES AMÉLIORÉES

(30) Priorität: 01.06.2011 DE 102011076869
(43) Veröffentlichungstag der Anmeldung: 09.04.2014
(73) Patentinhaber: Beiersdorf AG, 20253 Hamburg (DE)
(72) Erfinder: STORBECK, Celina, Bönningstedt 25474 (DE); KUMMER, Andreas, B., 21079 Hamburg (DE); SCHLENKER, David, 22525 Hamburg (DE)
(74) Vertreter: Hartmann, Jost
(86) Internationale Anmeldenummer: PCT/EP2012/060270
(87) Internationale Veröffentlichungsnummer: WO 2012/164030

(56) Entgegenhaltungen:
- EP-A1- 1 291 007
- EP-A1- 2 174 650
- EP-A2- 0 750 899
- WO-A2-2004/100862
- WO-A2-2012/012857
- DE-A1- 10 252 235
- DE-A1- 10 361 568

## Beschreibung

Die Erfindung beschreibt Emulsionszubereitungen umfassend ein oder mehrere emulgierende Polymere und ein oder mehrere hydroxyfunktionaliserte Verbindungen. Die Emulsionen zeigen spezifische rheologische Eigenschaften und lassen den "Wackelpudding-Effekt" ohne Zusatz an weiteren Emulgatoren verschwinden.

Als kosmetische oder medizinische Zubereitungen sind oftmals Emulsionen, insbesondere W/O-, O/W- oder W/O/W-Emulsionen, im Einsatz. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten (W/O) in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Der Stand der Technik kennt mehrere wesentliche Faktoren, die einen positiven Einfluss auf die Stabilität und Rheologie von Emulsionen haben:
1. feinste Verteilung der Phasen ineinander: Je feiner die eine Phase in der anderen verteilt ist, je kleiner damit die dispergierten Teilchen sind, umso stabiler ist die Emulsion.
2. möglichst geringer Unterschied in der Dichte der beiden Phasen.
3. ein ausgewogenes Phasenvolumenverhältnis.

Mehrkomponentensysteme wie Lösungen, Emulsionen oder Suspensionen werden häufig aus ökonomischen oder anwendungstechnischen Gründen oder aus Stabilitätsgründen auf höhere Viskositäten eingestellt bzw. verdickt.
So kann z.B. durch Erhöhung der Viskosität der externen oder internen Phase von Emulsionen oder Suspensionen erreicht werden, dass die Zeit bis zur Entmischung der Komponenten eines solchen Systems deutlich verlängert werden kann, was sich in einer Verlängerung der Lagerzeit bemerkbar macht. Durch Erhöhung der Viskosität wird auch bei vielen Produkten deren gleichmäßige Verteilbarkeit insbesondere auf unebenen Flächen verbessert. Dies gilt insbesondere für Hautpflegemittel und pharmazeutische Salben auf der Haut.

Das Temperaturverhalten der Polymere ist dabei eine wichtige Eigenschaft. Im Allgemeinen zeigen Polymere bei niedrigen Temperaturen eine hohe Viskosität, und bei hohen Temperaturen eine niedrige Viskosität. Erwünscht sind oftmals aber solche Polymere, die oberhalb bestimmter Temperaturen verdickend wirken, aber bei niedrigen Temperaturen in Lösung pumpbar und verarbeitbar bleiben, d.h. auch anwendungsfreundlich sind. Bekannt als derartige Verdicker sind beispielsweise Celluloseether und andere Cellulosederivate (z.B. Carboxymethylcellulose, Hydroxyethylcellulose), Gelatine, Stärke und Stärkederivate, Natriumalginate, Fettsäurepolyethylenglykolester, Agar-Agar, Traganth oder Dextrine.

Um Wasser und Öl stabil zu formulieren wird ein emulgierendes Polymer benötigt, wie beispielsweise das Acrylates/C10-30 Alkyl Acrylate Crosspolymer. Es zeigte sich, dass derartige Formulierungen einen "Wackelpudding-artigen" Charakter bekommen. Diese Zubereitung werden, ähnlich wie "Götterspeise", blockartig starr statt zähflüssig-cremig. Die Entnahme aus dem Vorratsbehältnis und die Verteilung auf dem Körper gestaltet sich als schwierig, da die Formulierung eine reduzierte Haftung auf der Haut aufweist. Sie "bröselt" eher auseinander statt sich glatt verstreichen zu lassen.

Aufgrund dieser negativen Eigenschaften bevorzugen Kosmetiker den Einsatz von Emulgatoren wie beispielsweise Glyceryl Stearate Citrate (Imwitor®), PEG- 40 Stearate, Polyglyceryl-3 Methylglucose Distearate (TC45) oder andere O/W Emulgatoren.

Emulsionen benötigen zu ihrer Bildung und zur Stabilisierung im Allgemeinen einen oder mehrere Emulgatoren, Verdicker und/oder Konsistenzgeber, um über einen kosmetisch akzeptablen Zeitraum stabil zu sein, im Allgemeinen 1 Jahr nach dem Öffnen einer kosmetischen Zubereitung.
Dazu werden oft große Mengen an Emulgatoren benötigt. Dies wiederum kann bei Konsumenten zu Missempfindungen bis zur Unverträglichkeit und im Extremfall sogar Phänomene wie die Mallorca Akne o.ä. führen.

Wünschenswert ist es daher Emulsionszubereitungen zur Verfügung zu stellen, die möglichst geringe Mengen an Emulgatoren umfassen und dennoch ausreichend stabil formuliert sind.

Wünschenswert ist es auch Formeln ohne Emulgator bereitzustellen, um ggf. die Verträglichkeit zu verbessern und andere sensorische Felder zu erschliessen.

EP0750899 offenbart Emulsionen, die polymerische Emulgatoren enthalten und keine weiteren emulgierenden Verbindungen. Die Erfindung ist eine kosmetische oder dermatologische Emulsionszubereitung umfassend ein oder mehrere emulgierende Polymere und ein oder mehrere hydroxyfunktionalisierte Verbindungen ausgewählt aus der Gruppe Hydroxybezaldehyde, Ethanol, Benzylalkohol, Methyl-1,3-propandiol, Hexan-1,2-diol und 2-Propanol im Anteil von 0,01 bis 0,6 Gew.% bezogen auf die Gesamtmasse der Zubereitung. Der Anteil an emulgierenden Polymeren beträgt mindestens 0,3 Gew.%, da unterhalb dieses Bereiches die resultierenden Zubereitungen sensorisch weniger attraktiv und die Viskosität sich als zu gering erweist.
Oberhalb von 0,3 % an emulgierenden Polymer, insbesondere Acrylates/C10-30 Alkyl Acrylate Crosspolymer, zeigt sich aber der zu vermeidende Wackelpudding-Effekt. Je höher der Anteil des Crosspolymers ist desto höher der Wackelpudding-effekt. Der Anteil an emulgierenden Polymer beträgt bis ca. 1 Gew.%, bezogen auf die Gesamtmasse der Zubereitung. Die Emulsionen zeigen spezifische rheologische Eigenschaften und lassen den "Wackelpudding-Effekt" ohne Zusatz an weiteren Emulgatoren verschwinden.

Die erfindungsgemäße Zubereitung umfasst daher bevorzugt des Weiteren keine weiteren emulgierenden Verbindungen. Geringe Mengen, unterhalb 0,1 Gew.% an emulgierenden Stoffen sind erfindungsgemäß akzeptabel und die erfindungsgemäße Zubereitungen sind dann dennoch als emulgatorfrei zu bezeichnen.

Die Emulsionszubereitungen sind bevorzugt Öl in Wasser Emulsionen. Die O/W Emulsion ist bevorzugt, weil sie einen größt möglichen sensorischen Spielraum bei der Entwicklung kosmetischer Zubereitung bietet.

Bei einer W/O ist die Sensorik vor allem durch die aussen liegenden Ölphase geprägt.

Als emulgierende Polymere sind vorteilhaft solche Systeme einzusetzen, die aufgrund ihrer molekularen Ausprägungen als Assoziativverdicker wirken können. Dieses sind beispielsweise hydrophobmodifizierte , ethoxylierte Urethane, hydrophobmodifizierte Cellulosether sowie hydrophob modifizierte (Meth)acyrlate. Letztgenannte Gruppe ist insbesondere zu wählen, sofern die hydrophoben Einheiten durch Alkygruppen mit einer Kettenlänge von C-10 bis C-30 gebildet werden, die entweder direkt an die C-C-Hauptkette gebunden oder mittels beispielsweise eines Spacers aus mehreren sich wiederholenden Einheiten an das Polymer gebunden sind. Diese Spacer entstammen dabei bevorzugt aus der Klasse der Polyether mit Monomereinheiten wie 1,2-Epoyxethan, 1,2-Epoxypropan oder Propan-1,2,3-triol.

Als emulgierende Polymere sind vorteilhaft Polyacrylsäure Verdicker, insbesondere das Acrylates/C10-30 Alkyl Acrylate Crosspolymer, zu wählen.

Acrylates/C10-30 Alkyl Acrylate Crosspolymer ist ein Copolymer von C10-30 Alkyacrylat und ein oder mehrere Monomeren der Acrylsäure, Methacrylsäure und deren einfacher Ester verzweigt mit einem Allylether von Sucrose oder einem Allylether von Pentaerythritol.

Das Acrylates/C10-30 Alkyl Acrylate Crosspolymer ist unter Acritamer 501ED (Rita), Acritamer 505ED (Rita), Aqupec HV-501ER (Sumitomo Seika Chemicals Co.), Carbopol ETD 2020 Polymer (Noveon), Carbopol 1342 Polymer (Noveon), Carbopol 1382 Polymer (Noveon), Carbopol Ultrez 20 Polymer (Noveon), Carbopol Ultrez 21 Polymer (Noveon), Pemulen TR-1 Polymer (Noveon), Pemulen TR-2 Polymer (Noveon) im Handel erhältlich.

Der Anteil an emulgierenden Polymeren, insbesondere Polyacrylsäure Verdicker, wie insbesondere Acrylates/C10-30 Alkyl Acrylate Crosspolymer, liegt im Bereich von 0,3 Gew.% bis zu 1 Gew.% bezogen auf die Gesamtmasse der Emulsion. Vorteilhaft liegt der Anteil an emulgierenden Polymeren im Bereich 0,3 bis 0,5 Gew.%.

Die erfindungsgemäße Emusion umfasst bevorzugt keine weiteren emulgierenden Polymere neben den Acrylates/C10-30 Alkyl Acrylate Crosspolymer.

Kosmetische oder dermatologische Formulierung ohne Emulgator sind bevorzugt, um nun aber Wasser und Öl stabil zu formulieren wird ein emulgierendes Polymer benötigt.

Um auch ausreichende Viskositäten einstellen zu können, wie beispielsweise Lotionen mit einer Viskosität von 3500 - 7000 mPas, müssen die Anteile des Polymers oberhalb von 0,3 Gew.% gewählt werden.

Nun hat sich aber in Versuchen herausgestellt, dass diese Formulierungen mit einem Anteil von 0,3% und mehr des emulgierenden Polymers einen Wackelpudding-ähnlichen Charakter haben.

Überraschenderweise wurde dann in folgenden Untersuchungen festgestellt, dass der Wackelpudding-Charakter beim Einsatz von 4- Hydroxybenzaldehyd (0,03%) nicht aufgetreten ist.

In weiteren Untersuchungen wurde der gleiche Effekt, das Nichtauftreten des Wackelpudding-Effektes, beim Einsatz von Ethanol, Benzylalkohol, Methylpropandiol, Hexandiol und Isopropylalkohol festgestellt.

Die offensichtliche Gemeinsamkeit der Rohstoffe, die für das Ausbleiben dieses Effektes verantwortlich ist, ist eine Hydroxyfunktionaliserung im Molekül.

Es zeigte sich, dass diese Hydroxyfunktionalität für den Rückgang des Wackelpudding-Effektes verantwortlich ist.

In weiteren Tests zeigte sich, dass Triole, wie Glycerin, den Effekt nicht zeigen. Ebenso zeigte Phenoxyethanol den Effekt nicht und der Wackelpudding-Effekt blieb.

Als erfindungsgemäße hydroxyfunktionalisierte Verbindungen kommen hierfür einerseits Moleküle mit mindestens einer und maximal zwei Hydroxyfunktionalitäten in Frage, wobei die an mindestens eine der Hydroxylgruppen gebundene Kohlenstoffkette mindestens zwei und höchstens acht Kohlenstoffatomen aufweist.

Andererseits sind als hydroxyfunktionalisierte Verbindungen Moleküle zu wählen mit mindestens einer und maximal zwei Hydroxyfunktionalitäten, wobei eine Hydroxylgruppe direkt an einen Phenylring gebunden ist, der mindestens einen Substituenten in gegenüberliedender Stellung zur Hydroxylgruppe besitzt, welcher dadurch gekennzeichnet ist, dass der Substituent mit der Phenylgruppe mit einem Kohlenstoffatom verbunden ist, welches seinerseits nicht mit einer weiteren Hydroxylgruppe verbunden ist.

Als hydroxyfunktionalisierte Verbindungen sind erfindungsgemäß zu wählen Hydroxybenzaldehyde, Ethanol, Benzylalkohol, Methyl-1,3-propandiol, Hexan-1,2-diol und 2-Propanol. Bevorzugt werden ein oder mehrere Hydroxybenzaldehyde gewählt aus der Gruppe, 3,4-Dihydroxybenzaldehyd, 4-Hydroxy-3-methoxybenzaldehyd (Vanilline), 4-Hydroxybenzaldehyd, 2,4-Dihydroxybenzaldehyd und/oder 4-Hydroxy-2-methoxybenzaldehyd, bevorzugt ist 4-Hydroxybenzaldehyd

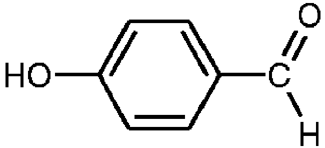

Als eine bevorzugte Ausführungsform lassen sich die erfindungsgemäßen Hydroxybenzaldehyde als Mischung mit Dipropylenglycol (CAS 25265-71-8), 1,2-Propylenglycol (CAS 57-55-6) und/oder den Verbindung II1 bzw. II2 (CAS 26330-65-4), wie sie in der EP 1029841 beschrieben werden, verwenden.

Vorteilhaft ist neben der Hydroxyfunktionalität, dass die Verbindungen über eine maximale räumliche Ausdehnung verfügen, die der des Hohlraumvolumens von gamma-Cyclodextrin (ca. 510 Å³) entspricht.

Das Verschwinden des Wackelpuddingeffektes beruht darauf, dass die Moleküle der hydroxyfunktionalisierten Verbindungen den Aufbau des 3D Netzwerkes der emulgierenden Polymere stören.

Der Anteil an hydroxyfunktionalisierten Verbindungen beträgt 0,01 bis 0,6 Gew.%, vorzugsweise 0,03 bis 0,3 Gew.%, bezogen auf die Gesamtmasse der Zubereitung.

Erfindungsgemäß ist der Anteil sowohl für die Gesamtmasse an hydroxyfunktionlisierten Verbindungen zu sehen als auch wenn nur eine der erfindungsgemäßen Verbindungen enthalten ist.

Bei Einschränkungen auf bevorzugte Verbindungen bleibt die Gesamtmenge an hydroxyfunktionalisierten Verbindungen jedoch als erfindungsgemäß vorteilhafte Maximalmenge bestehen.

In Vergleichsuntersuchungen wurde der Wackelpudding-Effekt rheologisch quantifiziert.

Untersucht wurden zunächst vier verschiedene kosmetische Zubereitungen gemäß nachfolgender Tabelle 1.

**Tabelle 1:**

| | 66 | 78 | 67 | 96 |
|---|---|---|---|---|
| INCI | m [%] | m [%] | m [%] | m [%] |
| Phenoxyethanol | 0,6 | 0,6 | 0,6 | 0,6 |
| Benzethonium Chloride | 0,07 | 0,07 | - | - |
| Glycerin | 9 | 9 | 9 | 9 |
| Octyldodecanol | 10 | 10 | 10 | 10 |
| Parfum | 0,3 | 0,3 | 0,3 | 0,3 |
| Sodium- Acrylates/C10-30 Alkyl Acrylate Crosspolymer | 0,4 | 0,4 | 0,4 | 0,4 |
| 4-Hydroxy Benzaldehyd | - | - | 0,03 | 0,03 |
| Aqua ad | 100 | 100 | 100 | 100 |

Die Zubereitungen 66 und 78 stellen dabei Zubereitungen ohne hydroxyfunktionalisierte Verbindungen und mit Polyacrylat-Crosspolymeren dar, 67 und 96 sind erfindungsgemäße Zubereitungen mit der erfindungsgemäße Kombination.

Für die Charakterisierung der Probezubereitungen wurde folgendes rheologisches Testverfahren ausgewählt.

G' wird auch als elastischer Anteil der Probe bezeichnet. Je höher dieser elastische Anteil (G') ist, desto wackelpuddingähnlicher erscheint auch die Emulsion, denn je stärker die elastischen Strukturen in einer Emulsion sind (höhere G'-Werte) desto stärker kann die Emulsion nach einer Belastung zurückfedern, desto eher gleicht sie einem Wackelpudding.

Auch das Verhältnis von Verlustmodul G" zu Speichermodul G' ist ausschlaggebend. Dieses wird als tan delta bezeichnet. Die tan delta Werte müssen über den gesamten gemessenen Frequenzbereich unterhalb von 0,3 liegen, damit ist sichergestellt, dass der Speichermodul G' deutlich überwiegt und damit auch die Emulsion überwiegend elastische (Wackelpudding artige) Eigenschaften besitzt. G' sagt dann aus, wie fest oder wie stark diese Eigenschaften sind.
Tan delta <1 fest, tan delta >1 fließfähig/ viskos

| | | |
|---|---|---|
| Methode: | Gerät: | ARES - Hersteller: Rheometrix Cientific |
| | Messung: | Frequenztest |
| | Meßtemp.: | 25°C Peltiertemperierung |
| | Meßsystem: | Platte-Platte 25mm Durchmesser, 1mm Spalt |
| | Programm: | 0,1 → 100 rad/s |

Danach zeigen die getesteten Zubereitungen ein elastischen Anteil (G') von

| Tabelle 2 | G' |
|---|---|
| Probe | 1 [rad/s] |
| -66- | 252 |
| -67- | 108 |
| -78- | 314 |
| -96- | 137 |

Erfindungsgemäße Zubereitungen sind demnach diejenigen mit einem G' < 137 [rad/s] gemessen nach der Frequenzmethode.
Dieser rheologische Effekt einer Zubereitung mit einem G' größer 137 rad/s wird erfindungsgemäß als "Wackelpudding-Effekt" bezeichnet

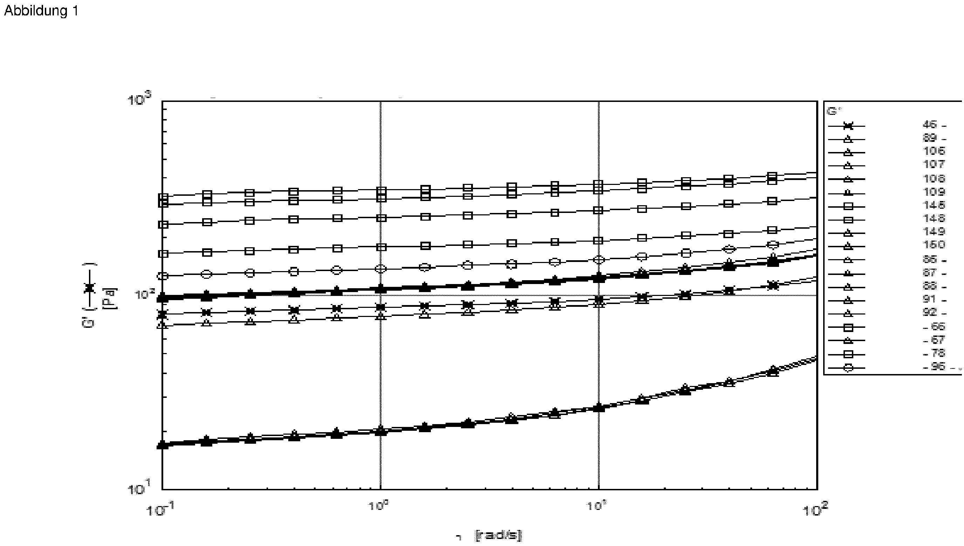

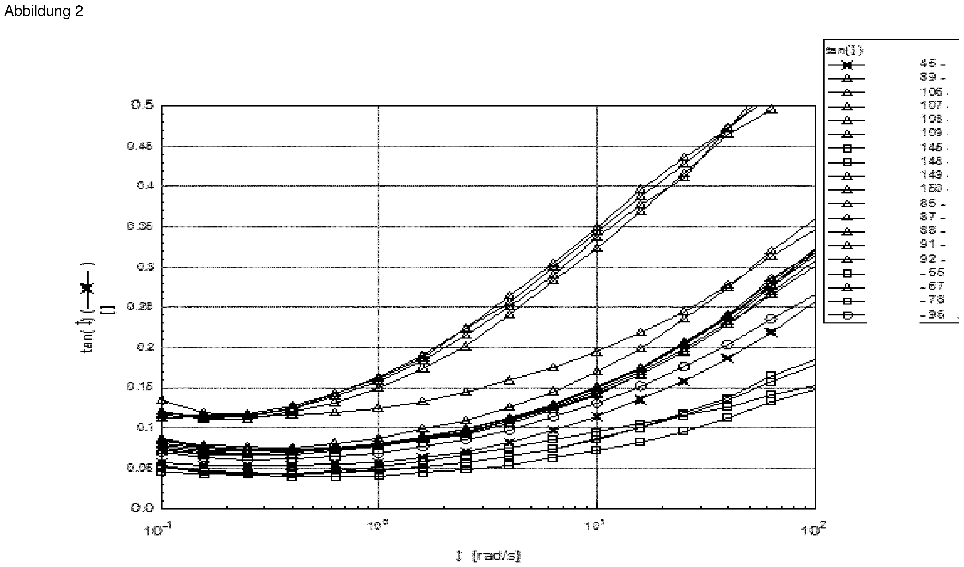

Abbildungen 1 und 2 zeigen den elastischen Anteil (G') bzw. tan delta weiterer untersuchter Zubereitungen.
Der mit einem Kreis gekennzeichnete Graph (ONLYX 96) in den Abbildungen stellt die Grenze von 137 rad/s dar. Unterhalb dieses Wertes zeigten sich keine Wackelpuddingeffekte. Die Rezeptur mit einem Stern ist ohne hydroxyfunktionalisierte Moleküle und ohne Wackelpuddingeffekt. Die Beispiele mit Dreiecksymbolen stellen erfindungsgemäße Zubereitungen ohne einen Wackelpuddingeffekt dar, die Beispiele mit Quadratsymbolen stellen Zubereitungen mit einem Wackelpuddingeffekt dar.
Die getesteten Rezepturen sind in den nachfolgenden Tabellen 3 angeführt.

| | Zubereitungen mit Wackelpuddingeffekt | | | |
|---|---|---|---|---|
| | 66 | 78 | 145 | 148 |
| INCI | m [%] | m [%] | m [%] | m [%] |
| Sodium- Acrylates/C10-30 Alkyl Acrylate Crosspolymer | 0,4 | 0,4 | 0,4 | 0,3 |
| Octyldodecanol | 10 | 10 | 10 | 10 |
| Glycerin | 9 | 9 | 9 | 9 |
| Benzethonium Chloride | 0,07 | 0,07 | 0,07 | 0,07 |
| Phenoxyethanol | 0,6 | 0,6 | 0,6 | 0,6 |
| Parfum | 0,3 | 0,3 | 0,3 | 0,3 |
| Aqua ad | 100 | 100 | 100 | 100 |

| | Zubereitungen ohne Wackelpuddingeffekt | | | | | |
|---|---|---|---|---|---|---|
| | 46 | 67 | 86 | 87 | 88 | 89 |
| INCI | m [%] | m [%] | m [%] | m [%] | m [%] | m [%] |
| Sodium- Acrylates/C10-30 Alkyl Acrylate Crosspolymer | 0,2 | 0,4 | 0,4 | 0,4 | 0,4 | 0,4 |
| 4-Hydroxy Benzaldehyd | | 0,03 | | | | |
| Methylparaben | | | 0,3 | | | |
| Benzyl Alcohol | | | | | | 0,3 |
| Isopropyl Alcohol | | | | | 0,3 | |
| Alcohol Denat. | | | | 0,3 | | |
| Octyldodecanol | 10 | 10 | 10 | 10 | 10 | 10 |
| Glycerin | 9 | 9 | 9 | 9 | 9 | 9 |
| Benzethonium Chloride | 0,07 | | | | | |
| Phenoxyethanol | 0,6 | 0,6 | 0,6 | 0,6 | 0,6 | 0,6 |
| Parfum | 0,3 | 0,3 | 0,3 | 0,3 | 0,3 | 0,3 |
| Aqua ad | 100 | 100 | 100 | 100 | 100 | 100 |

| | Zubereitungen ohne Wackelpuddingeffekt | | | | |
|---|---|---|---|---|---|
| | 91 | 92 | 96 | 106 | 107 |
| INCI | m [%] | m [%] | m [%] | m [%] | m [%] |
| Sodium- Acrylates/C10-30 Alkyl Acrylate Crosspolymer | 0,4 | 0,4 | 0,4 | 0,4 | 0,4 |
| 4-Hydroxy Benzaldehyd | | | 0,03 | 0,03 | |
| Methylpropanediol | | 0,3 | | | 0,07 |
| 1,2-Hexanediol | 0,3 | | | | |
| Octyldodecanol | 10 | 10 | 10 | 10 | 10 |
| Glycerin | 9 | 9 | 9 | 9 | 9 |
| Phenoxyethanol | 0,6 | 0,6 | 0,6 | 0,6 | 0,6 |
| Parfum | 0,3 | 0,3 | 0,3 | 0,3 | 0,3 |
| Aqua ad | 100 | 100 | 100 | 100 | 100 |

| | Zubereitungen ohne Wackelpuddingeffekt | | | |
|---|---|---|---|---|
| | 108 | 109 | 149 | 150 |
| INCI | m [%] | m [%] | m [%] | m [%] |
| Sodium- Acrylates/C10-30 Alkyl Acrylate Crosspolymer | 0,4 | 0,4 | 0,4 | 0,4 |
| 4-Hydroxy Benzaldehyd | | | 0,02 | 0,01 |
| Methylparaben | | 0,07 | | |
| Alcohol Denat. | 0,07 | | | |
| Octyldodecanol | 10 | 10 | 10 | 10 |
| Glycerin | 9 | 9 | 9 | 9 |
| Phenoxyethanol | 0,6 | 0,6 | 0,6 | 0,6 |
| Parfum | 0,3 | 0,3 | 0,3 | 0,3 |
| Aqua ad | 100 | 100 | 100 | 100 |

Zubereitungen 86 und 109 sind nicht erfindungsgemäß. Durch den Zusatz der hydroxyfunktionalisierten Moleküle, insbesondere des Benzaldehyds, verschwindet der Wackelpudding-Effekt, die Emulsion ist in sich geschlossener, sie fließt gleichmäßiger und verhält sich nicht stückhaft wie Wackelpudding.

Die erfindungsgemäße Textur äußert sich in einer für den Anwender äußerst angenehmen Applikations- und Anwendungsweise, die Emulsion hat ein gleichmäßiges Fließverhalten und ist auf der Haut gut und gleichmäßig verteilbar vor allem ist die Emulsion ohne diesen Wackelpudding Charakter besser abzufüllen.

Es ist dem Fachmann natürlich bekannt, dass anspruchsvolle kosmetische oder dermatologische Zusammensetzungen zumeist nicht ohne die üblichen Hilfs- und Zusatzstoffe formulierbar sind. Darunter zählen beispielsweise Konsistenzgeber, Füllstoffe, Parfum, Farbstoffe, zusätzliche Wirkstoffe wie Vitamine oder Proteine, Lichtschutzmittel, Stabilisatoren, Insektenrepellentien, Alkohol, Wasser, Salze, Substanzen zum Verhindern des Schäumens, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie organische Lösungsmittel, Silikonderivate oder Moisturizer usw. als auch antimikrobiell, proteolytisch oder keratolytisch wirksame Substanzen, Konservierungsmittel, Fungizide oder Bakterizide sofern sie nicht explizit ausgenommen sind oder die erfindungsgemäßen rheologischen Eigenschaften verhindern.

Nachfolgende Beispielzubereitungen erläutern die erfindungsgemäßen Emulsionen. Die aufgeführten Zahlenwerte stellen Gewichtsanteile bezogen auf die jeweilige Gesamtmasse der Zubereitungen dar. Die Zubereitung E ist nicht erfindungsgemäß.

### Beispiele

| | A | B | C | D | E | F |
|---|---|---|---|---|---|---|
| INCI | m [%] | m [%] | m [%] | m [%] | m [%] | m [%] |
| Sodium- Acrylates/C10-30 Alkyl Acrylate Crosspolymer | 0,5 | 0,7 | 0,65 | 0,8 | 0,4 | 0,4 |
| 4-Hydroxy Benzaldehyd | 0,03 | | 0,03 | 0,03 | | 0,02 |
| Caprylic/ Capric Triglyceride | 2 | 4 | 4,5 | | 6 | |
| Dicaprylyl Carbonate | | 5 | | 5,5 | 2 | |
| Isopropylpalmitat | 8 | 2 | | 5 | 3 | 3 |
| Octyldodecanol | | | 5 | 2 | | |
| Glycerin | 9 | 9 | 4 | 9 | 6 | 6 |
| Cyclomethicon | | | 1 | | | 5 |
| Dimethicone | 1 | | | | | 1 |
| Methylparaben | 0,3 | | | | 0,4 | |
| Methylpropanediol | | | 0,27 | 0,5 | | |
| 1,2-Hexanediol | | 0,4 | | | | |
| Phenoxyethanol | 0,6 | 0,6 | 0,6 | 0,6 | 0,6 | 0,6 |
| Parfum | 0,3 | 0,3 | 0,3 | 0,3 | 0,3 | 0,3 |
| Aqua ad | 100 | 100 | 100 | 100 | 100 | 100 |

## Patentansprüche

1. Kosmetische oder dermatologische Emulsionszubereitung umfassend ein oder mehrere emulgierende Polymere und ein oder mehrere hydroxyfunktionalisierte Verbindungen **dadurch gekennzeichnet, dass**
▪ der Anteil an emulgierenden Polymeren mindestens 0,3 Gew.% bis zu 1 Gew.%, bezogen auf die Gesamtmasse der Zubereitung, beträgt,
▪ der Anteil an weiteren emulgierenden Verbindungen unter 0,1 Gew.% beträgt und
▪ ein oder mehrere hydroxyfunktionalisierte Verbindungen gewählt werden aus der Gruppe Hydroxybenzaldehyde, Ethanol, Benzylalkohol, Methyl-1,3-propandiol, Hexan-1,2-diol und 2-Propanol und deren Anteil im Bereich von 0,01 bis 0,6 Gew.%, gewählt wird, jeweils bezogen auf die Gesamtmasse der Zubereitung

2. Zubereitung nach Anspruch 1 **dadurch gekennzeichnet, dass** die Zubereitung eine Öl in Wasser (O/W) Emulsion ist.

3. Zubereitung nach einem der vorstehenden Ansprüche **dadurch gekennzeichnet, dass** als emulgierendes Polymer Acrylates/C10-30 Alkyl Acrylate Crosspolymer gewählt wird.

4. Zubereitung nach einem der vorstehenden Ansprüche **dadurch gekennzeichnet, dass** der Anteil an emulgierenden Polymeren bis zu 0,5 Gew.%, bezogen auf die Gesamtmasse der Zubereitung, gewählt wird.

5. Zubereitung nach Anspruch 1 **dadurch gekennzeichnet dass** als Hydroxybenzaldehyde gewählt werden 3,4-Dihydroxybenzaldehyd, 4-Hydroxy-3-methoxybenzaldehyd (Vanilline), 4-Hydroxybenzaldehyd, 2,4-Dihydroxybenzaldehyd und/oder 4-Hydroxy-2-methoxybenzaldehyd.

6. Zubereitung nach einem der vorstehenden Ansprüche **dadurch gekennzeichnet, dass** der Anteil an hydroxyfunktionalisierten Verbindungen aus der Gruppe Hydroxybenzaldehyde, Ethanol, Benzylalkohol, Methyl-1,3-propandiol, Hexan-1,2-diol und 2-Propanol 0,03 bis 0,3 Gew.%, bezogen auf die Gesamtmasse der Zubereitung, beträgt.

7. Verwendung von hydroxyfunktionaliserten Verbindungen in kosmetischen oder dermatologischen Emulsionszubereitungen umfassend 0,3 bis 1 Gew.% an emulgierenden Polymeren und weniger als 0,1 Gew.% an weiteren emulgierenden Verbindungen zur Einstellung eines elastischen Anteils G' der Zubereitung von kleiner 137 [rad/s], wobei ein oder mehrere hydroxyfunktionalisierte Verbindungen gewählt werden aus der Gruppe Hydroxybenzaldehyde, Ethanol, Benzylalkohol, Methyl-1,3-propandiol, Hexan-1,2-diol und 2-Propanol und deren Anteil im Bereich von 0,01 bis 0,6 Gew.%, gewählt wird, jeweils bezogen auf die Gesamtmasse der Zubereitung.

## Claims

1. Cosmetic or dermatological emulsion preparation comprising one or more emulsifying polymers and one or more hydroxy-functionalized compounds, **characterized in that**
▪ the fraction of emulsifying polymers is at least 0.3% by weight up to 1% by weight, based on the total mass of the preparation,
▪ the fraction of further emulsifying compounds is less than 0.1% by weight and
▪ one or more hydroxy-functionalized compounds are selected from the group comprising hydroxybenzaldehyde, ethanol, benzyl alcohol, methyl-1,3-propanediol, hexane-1,2-diol and 2-propanol and the fraction thereof is selected in the range of 0.01 to 0.6% by weight, based in each case on the total mass of the preparation.

2. Preparation according to Claim 1, **characterized in that** the preparation is an oil-in-water (O/W) emulsion.

3. Preparation according to either of the preceding claims, **characterized in that** acrylates/C10-30 alkyl acrylate crosspolymer is selected as emulsifying polymer.

4. Preparation according to any of the preceding claims, **characterized in that** the fraction of emulsifying polymers is selected to an extent of up to 0.5% by weight, based on the total mass of the preparation.

5. Preparation according to Claim 1, **characterized in that** 3,4-dihydroxybenzaldehyde, 4-hydroxy-3-methoxybenzaldehyde (vanillin), 4-hydroxybenzaldehyde, 2,4-dihydroxybenzaldehyde and/or 4-hydroxy-2-methoxybenzaldehyde are selected as hydroxybenzaldehydes.

6. Preparation according to any of the preceding claims, **characterized in that** the fraction of hydroxy-functioualized compounds from the group comprising hydroxybenzaldehyde, ethanol, benzyl alcohol, methyl-1,3-propanediol, hexane-1,2-diol and 2-propanol is 0.03 to 0.3% by weight, based on the total mass of the preparation.

7. Use of hydroxy-functionalized compounds in cosmetic or dermatological emulsion preparations comprising 0.3 to 1% by weight of emulsifying polymers and less than 0.1% by weight of further emulsifying compounds for setting an elastic fraction G' of the preparation of less than 137 [rad/s], wherein one or more hydroxy-functionalized compounds are selected from the group comprising hydroxybenzaldehyde, ethanol, benzyl alcohol, methyl-1,3-propanediol, hexane-1,2-diol and 2-propanol and the fraction thereof is selected in the range of 0.01 to 0.6% by weight, based in each case on the total mass of the preparation.

## Revendications

1. Préparation d'émulsion cosmétique ou dermatologique comprenant un ou plusieurs polymères émulsifiants et un ou plusieurs composés à fonctiormalisation hydroxy, **caractérisée en ce que**
- la proportion de polymères émulsifiants est d'au moins 0,3 % en poids à 1 % en poids, par rapport à la masse totale de la préparation,
- la proportion de composés émulsifiants supplémentaires est inférieure à 0,1 % en poids, et
- un ou plusieurs composés à fonctionnalisation hydroxy sont choisis dans le groupe constitué par les hydroxybenzaldéhydes, l'éthanol, l'alcool benzylique, le méthyl-1,3-propanediol, l'hexane-1,2-diol et le 2-propanol, et leur proportion est choisie dans la plage allant de 0,01 à 0,6 % en poids, à chaque fois par rapport à la masse totale de la préparation.

2. Préparation selon la revendication 1, **caractérisée en ce que** la préparation est une émulsion huile dans eau (H/E).

3. Préparation selon l'une quelconque des revendications précédentes, **caractérisée en ce qu'**Acrylates/C10-30 Alkyl Acrylate Crosspolymer est choisi en tant que polymère émulsifiant.

4. Préparation selon l'une quelconque des revendications précédentes, **caractérisée en ce que** la proportion de polymères émulsifiants est choisie de jusqu'à 0,5 % en poids, par rapport à la masse totale de la préparation.

5. Préparation selon la revendication 1, **caractérisée en ce que** le 3,4-dihydroxybenzaldéhyde, le 4-hydroxy-3-méthoxybenzaldéhyde (vanilline), le 4-hydroxybenzaldéhyde, le 2,4-dihydroxybenzaldéhyde et/ou le 4-hydroxy-2-méthoxybenzaldéhyde sont choisis en tant qu'hydroxybenzaldéhydes.

6. Préparation selon l'une quelconque des revendications précédentes, **caractérisée en ce que** la proportion de composés à fonctionnalisation hydroxy du groupe constitué par les hydroxybenzaldéhydes, l'éthanol, l'alcool benzylique, le méthyl-1,3-propanediol, l'hexane-1,2-diol et le 2-propanol est de 0,03 à 0,3 % en poids, par rapport à la masse totale de la préparation.

7. Utilisation de composés à fonctionnalisation hydroxy dans des préparations d'émulsion cosmétiques ou dermatologiques comprenant 0,3 à 1 % en poids de polymères émulsifiants et moins de 0,1 % en poids de composés émulsifiants supplémentaires pour l'ajustement d'une fraction élastique G' de la préparation inférieure à 137 [rad/s], un ou plusieurs composés à fonctionnalisation hydroxy étant choisis dans le groupe constitué par les hydroxybenzaldéhydes, l'éthanol, l'alcool benzylique, le méthyl-1,3-propanediol, l'hexane-1,2-diol et le 2-propanol, et leur proportion étant choisie dans la plage allant de 0,01 à 0,6 % en poids, à chaque fois par rapport à la masse totale de la préparation.
